# EUROPEAN PATENT SPECIFICATION

(11) **EP 1 047 426 B1**
(45) Date of publication and mention of the grant of the patent: **12.05.2010**
(21) Application number: 98957573.3
(22) Date of filing: 03.11.1998
(51) Int. Cl.: A61K 31/485, A61P 1/00

(54) **USE OF METHYLNALTREXONE AND RELATED COMPOUNDS**
VERWENDUNG VON METHYLNALTREXON UND VERWANDTEN VERBINDUNGEN
UTILISATION DU METHYLNALTREXONE ET COMPOSES AFFERENTS A CE COMPOSE

(30) Priority: 03.11.1997 US 962742; 22.07.1998 US 120703
(43) Date of publication of application: 02.11.2000
(73) Proprietor: Arch Development Corporation, Chicago, IL 60637 (US); UR LABS, INC., Reno, Nevada 89501 (US)
(72) Inventor: DRELL, William, San Diego, CA 92122 (US); FOSS, Joseph, F., Chicago, IL 60613 (US); ROIZEN, Michael, F., Chicago, IL 60637 (US); MOSS, Jonathan, Chicago, IL 60637 (US); YUAN, Chun-Su, Chicago, IL 60615 (US)
(74) Representative: Jump, Timothy John Simon
(86) International application number: PCT/US1998/023485
(87) International publication number: WO 1999/022737

(56) References cited:
- EP-A- 0 306 575
- US-A- 4 719 215
- US-A- 4 861 781
- US-A- 5 102 887
- JAMES RUSSELL ET ALL.: "Antagonism of gut, but not central effects of morphine with quaternary narcotic antagonists" EUROPEAN JOURNAL OF PHARMACOLOGY, vol. 78, 1982, pages 255-261, XP002280199
- YUAN et al., "The Safety and Efficacy of Oral Methylnaltrexone in Preventing Morphine-Induced Delay in Oral-Cecal Transit Time", CLINICAL PHARMACOLOGY & THERAPEUTICS, April 1997, Vol. 61, Number 4, pages 467-475, XP002915586

## Description

### FIELD OF THE INVENTION

The present invention is in the field of treating non-opioid-induced inhibition of gastrointestinal motility.

### BACKGROUND OF THE INVENTION

Opioids are effective analgesics. However, their use is associated with a number of undesirable side effects. One of these side effects is pruritus, or itching. Pruritus is a common side effect associated with the use of opioids and may be very severe. Pruritus can occur when the opioid is administered intramuscularly, intravenously, transdermally, transmucosally or intrathecally.

It is believed that the opioid induced pruritus results from the release of histamine in response to the administration of opioids. Opioids are thought to stimulate histamine release by binding to opioid receptors on the central nervous system. This, in turn, causes peripheral nerves and histamine containing cells to release histamine.

Based on this theory a number of treatments have been used to alleviate opioid induced pruritus. The first is the use of antihistamines. However, antihistamines have a variable effect on opioid induced pruritus. Additionally, the use of antihistamines, when effective, only treats the symptom after it has occurred, rather than preventing its occurrence.

Another undesirable side effect of opioids is urinary retention, or the patient's inability to spontaneously empty his or her bladder. This urinary retention is a common side effect that can occur when opioids or related compounds are administered intramuscularly, intravenously, transmucosally, transdermally, or intrathecally. It is not clear why opioids cause urinary retention, but it is thought to be related to the central anticholinergic stimulation that opioids induce. Based on this theory, a number of cholinergic-type drugs have been used to treat urinary retention. However, due to the side effects of cholinergic drugs, catheterization of the bladder with a tube to drain urine remains the mainstay of treatment.

Another opioid-induced side effect is dysphoria, a feeling of unpleasantness or discomfort. Many subjects, especially those without pain, report unpleasant psychomimetic responses to the administration of an opioid alone. These responses have been previously attributed to activation of centrally located opioid receptors. This opioid-induced dysphoria is commonly treated by the addition of other drugs, such as benzodiazepines, to decrease the dysphoria or to blunt the recall of the dysphoria. These drugs, however are associated with increased levels of sedation and may enhance respiratory depression caused by the opioid.

Another side effect is constipation. Opioid-induced changes in gastrointestinal motility are almost universal when these drugs are used to treat pain, and at times may limit their use, leaving the patient in pain. Common treatments of bulking agents and laxatives have limited efficacy and may be associated with side effects such as electrolyte imbalances.

One treatment for side effects such as pruritis, urinary retention, dysphoria, and inhibited gastrointestinal motility is the use of opioid antagonists which cross the blood-brain-barrier, or which are administered directly into the central nervous system. Opioid antagonists such as naltrexone and naloxone have been administered intramuscularly or orally to treat opioid induced pruritus. Naltrexone and naloxone are highly lipid soluble and rapidly diffuse across biological membranes, including the blood-brain-barrier. However, naltrexone, naloxone and other opioid antagonists also reduce the analgesic effect of the opioid being used.

Many quaternary amine opioid antagonist derivatives, such as methylnaltrexone, do not reduce the analgesic effect of the opioids. These quaternary amine opioid antagonist derivatives, which have a relatively higher polarity and reduced lipid solubility when compared to the tertiary forms of the drugs, were specifically developed to not traverse the blood-brain-barrier or to traverse it at a greatly reduced rate. Since these quaternary opioid antagonist derivatives do not cross the blood-brain-barrier, peripheral administration of these antagonists would not be expected to be effective in the treatment of an opioid induced side effect caused by the opioid within the central nervous system. In fact, experiments show that to be effective in blocking the opioid receptors in the central nervous system, these antagonists must be injected directly into the central nervous system. However, injection of drugs directly into the central nervous system is undesirable since it increases the possibility of introducing bacterial or viral contamination to the central nervous system.

Yuan et al., writing in Clinical Pharmacology & Therapeutics, April 1997, vol. 61, No. 4, pages 467-475, have reported on the safety and efficacy of oral methylnaltrexone in preventing morphine-induced delay in oral-cecal transit time.

It is desirable in the treatment of many conditions to have oral medications with prolonged effects. Such oral medications are particularly desirable both for the treatment of nonopioid-induced side effects including constipation and delayed gastric emptying or inhibition of gastrointestinal motility caused by abdominal surgery.

It is further desirable to develop a method for the prevention of inhibition of gut motility, and constipation, which does not counteract the analgesic effects of an opioid, or risk increased levels of pain. Ideally, such a treatment has few side effects either due to low drug toxicity or because administration of small amounts are effective and/or administration results in low circulating levels of the drug.

### SUMMARY OF THE INVENTION

The present invention relates to compounds for use in preventing and treating nonopioid-induced inhibition of gastrointestinal motility.

According to the present invention, there is provided a quaternary derivative of noroxymorphone of the formula: 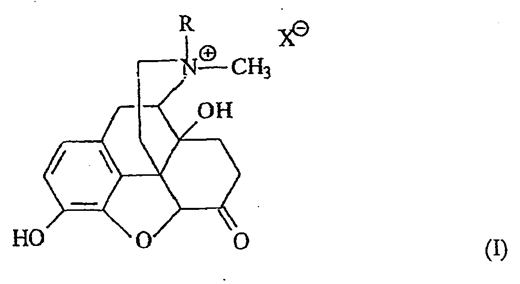 wherein R is allyl, chlorallyl, cyclopropyl-methyl or propargyl, and X is the anion of an acid, for treating inhibition of gastrointestinal motility caused by abdominal surgery.

Quaternary derivatives of noroxymorphone of formula I are disclosed in US Patent No. 4,176,186.

The methylnaltrexone or enteric coated methylnaltrexone, or other quaternary derivative of noroxymorphone, can be administered to a patient prior to or other the development of inhibited gastrointestinal motility and the route of administration can be intravenous, intramuscular, transmucosal, transdermal or oral administration in a standard or enterically coated preparation.

### DETAILED DESCRIPTION OF DRAWINGS

FIG. 1A is a graph representing plasma concentrations of MNTX following administration of 6.4 mg/kg of uncoated MNTX.
FIG. 1B is a graph representing plasma concentrations of MNTX following administration of 6.4 mg/kg of enterically coated MNTX.
FIG. 1C is a graph representing plasma concentrations of MNTX following administration of 3.2 mg/kg of enterically coated MNTX.
FIG. 2 illustrates the reversal of morphine's effect on oral-cecal transit time following administration of 6.4 mg/kg of uncoated MNTX. The darker line represents the average of all points of a given treatment.
FIG. 3 illustrates the reversal of morphine's effect on oral-cecal transit time and its decrease below baseline following administration of 6.4 mg/kg of enterically coated MNTX. The darker line represents the average of all points of a given treatment.
FIG. 4 illustrates the reversal of morphine's effect on oral-cecal transit time following administration of 3.2 mg/kg of enterically coated MNTX. The darker line represents the average of all points of a given treatment.

### DETAILED DESCRIPTION OF THE INVENTION

The present invention relates to compounds for use in to preventing and treating inhibition of gastrointestinal mobility caused by abdominal surgery.

The oral administration of non-enterically coated MNTX is associated with plasma levels with an early peak (20 min) and prolonged presence (half-life of about 3 hours after single dose of 6.4 mg/kg). However, an enteric coating on the QDNM, designed to prevent dissolution and subsequent absorption of the drug in the stomach, would be expected to produce delayed elevation of plasma levels of the QDNM, and to produce a lower peak plasma level. Suprisingly, however, administration of enterically coated MNTX has been found to result in substantially lower plasma levels as compared to non-enterically coated MNTX at the same dosage level, and surprisingly and unexpectedly resulted in enhanced efficacy in the reversal of opioid-induced decreases in gastrointestinal motility. In fact, it has been found that as compared to non-enterically coated MNTX, a significantly lower dose, e.g., less than half the amount of coated MNTX can be used if enterically coated to achieve the same levels of relief of opioid-induced constipation. Moreover, such reduced dosage levels of MNTX administered with an enteric coating results in exceedingly low peak and sustained plasma levels of MNTX, greatly reducing the potential adverse side effects of the MNTX. This novel improvement in the clinical indication for use of MNTX has led to an increased therapeutic index for this drug.

When used as a treatment for reduction of gastrointestinal motility, orally administered, particularly if enterically coated, MNTX or other quaternary derivatives of noroxymorphone provide prolonged relief.

Furthermore, for treatment or prevention of constipation and delayed gastrointestinal emptying, whether caused by extrinsic or endogenous opioids, enteric coating surprisingly allows for equal or better efficacy despite lower plasma levels. Idiopathic constipation, i.e., that due to causes other than exogenous administration of opioids, may be mediated by opioid sensitive mechanisms. Endogenous opioid receptors have been identified in the gut, and these receptors may modulate gut motility. Thus, administration of an opioid antagonist with peripheral action, such a methylnaltrexone or other quaternary derivatives of noroxymorphone, would block the effects of endogenous opioids.

Quaternary derivatives of noroxymorphone are described in full in U.S. Patent No. 4,176,186, and in general are represented by the formula: 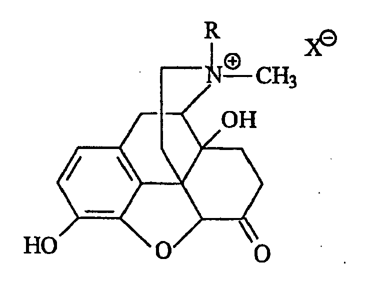 wherein R is allyl or a related radical such as chlorallyl, cyclopropyl-methyl or propargyl, and X is the anion of an acid, especially a chloride, bromide, iodide or methylsulfate anion.

The presently preferred quaternary derivative of noroxymorphone is methylnaltrexone. Methylnaltrexone is a quaternary amine derivative of naltrexone. Methylnaltrexone has been found to have only 2 to 4% of the opiate antagonistic activity of naltrexone *in vivo* due to its inability to pass the blood-brain-barrier and bind to the opiate receptors in the central nervous system.

Opioids are typically administered at a morphine equivalent dosage of: 0.005 to 0.15 mg/kg body weight for intrathecal administration; 0.05 to 1.0 mg/kg body weight for intravenous administration; 0.05 to 1.0 mg/kg body weight for intramuscular administration; 0.05 to 1.0 mg/kg body weight/hour for transmucosal or transdermal administration. By "morphine equivalent dosage" is meant representative doses of other opioids which equal one milligram of morphine, for example 10 mg meperidine, 1 mg methadone, and 80 µg fentanyl.

In accordance with the present invention, methylnaltrexone is administered at a dosage of: 0.03 to 1.0 mg/kg body weight for intravenous administration; 0.03 to 1.0 mg/kg body weight for intramuscular administration; 0.03 to 1.0 mg/kg body weight for transmucosal administration and 0.1 to 80.0 mg/kg body weight for oral administration, including enterically coated methylnaltrexone.

It is preferable to administer the methylnaltrexone prior to the onset of inhibition of gastrointestinal motility, in order to prevent its symptoms from manifesting.

Methylnaltrexone is rapidly absorbed after oral administration from the stomach and bowel. Initial plasma levels of the drug are seen within 5-10 minutes of the administration of non-enteric coated compound. Addition of an enteric coating which prevents gastric absorption is associated with lower plasma levels of the methylnaltrexone. Surprisingly, the addition of an enteric coating (i.e., a coating which will prevent degradation or release in the stomach, but will release drug in the small and large bowel) enhances the efficacy of methylnaltrexone in the prevention of decreases in gut motility by intravenously administered opioids (morphine).

For intravenous administration, methylnaltrexone is formulated with saline or other physiologically acceptable carriers; for intramuscular administration, the methylnaltrexone is formulated with saline or other pharmacologically acceptable carriers; for transmucosal administration the methylnaltrexone is formulated with a sugar and cellulose mix or other pharmacologically acceptable carriers known in the art; and for oral administration, the methylnaltrexone is formulated with pharmacologically acceptable binders to make a tablet or capsule with or without an enteric coating. Methods for such formulations are well known to those skilled in the art.

In a preferred embodiment for the prevention and/or treatment of constipation and inhibition of gastrointestinal motility, the QDNM or MNTX is enterically coated and administered orally. For oral administration, the QDNM or methylnaltrexone is formulated with pharmacologically acceptable binders to make a tablet or capsule with an enteric coating. An enteric coating is one which remains intact during passage through the stomach, but dissolves and releases the contents of the tablet or capsule once it reaches the small intestine. Most currently used enteric coatings are those which will not dissolve in low pH environments, but readily ionize when the pH rises to about 4 or 5, for example synthetic polymers such as polyacids having a pKₐ of 3 to 5.

The enteric coating may be made of any suitable composition. Suitable enteric coatings are described, for example, in U.S. Patent Nos. 4,311,833 to Namikoshi, et al.; 4,377,568 to Chopra; 4,385,078 to Onda, et al.; 4,457,907 to Porter; 4,462,839 to McGinley, et al.; 4,518,433 to McGinley, et al.; 4,556,552 to Porter, et al.; 4,606,909 to Bechgaard et al.; 4,615,885 to Nakagame, et al.; 4,670,287 to Tsuji; 5,536,507 to Abramowitz, et al.; 5,567,423 to Ying, et al.; 5,591,433 to Michael, et al.; 5,597,564 to Ying, et al.; 5,609,871 to Michael, et al.; 5,614,222 to Kaplan; 5,626,875 to Rodes, et al.; and 5,629,001 to Michael, et al..

Preferred enteric coating compositions include alkyl and hydroxyalkyl celluloses and their aliphatic esters, e.g., methylcellulose, ethylcellulose, hydroxyethylcellulose, hydroxypropylcellulose, hydroxybutylcellulose, hydroxyethylethylcellulose, hydroxyprophymethylcellulose, hydroxybutylmethylcellulose, hydroxypropylcellulose phthalate, hydroxypropylmethylcellulose phthalate and hydroxypropylmethylcellulose acetate succinate; carboxyalkylcelluloses and their salts, e.g., carboxymethylethylcellulose; cellulose acetate phthalate; cellulose acetate trimellitate, polycarboxymethylene and its salts and derivatives; polyvinyl alcohol and its esters: polyvinyl acetate phthalate; polycarboxymethylene copolymer with sodium formaldehyde carboxylate; acrylic polymers and copolymers, e.g., methacrylic acid-methyl methacrylic acid copolymer and methacrylic acid-methyl acrylate copolymer; edible oils such as peanut oil, palm oil, olive oil and hydrogenated vegetable oils; polyvinylpyrrolidone; polyethylene glycol and its esters: natural products such as shellac, and zein.

Other preferred enteric coatings include polyvinylacetate esters, e.g., polyvinyl acetate phthalate; alkyleneglycolether esters of copolymers such as partial ethylene glycol monomethylether ester of ethylacrylate-maleic anhydride copolymer or diethyleneglycol monomethylether ester of methylacrylate-maleic anhydride copolymer, N-butylacrylate-maleic anhydride copolymer, isobutylacrylate-maleic anhydride copolymer or ethylacrylate-maleic anhydride copolymer; and polypeptides resistant to degradation in the gastric environment, e.g., polyarginine and polylysine. Other suitable coatings and methods to make and use such formulations are well known to those skilled in the art (see, e.g., Remington: The Science and Practice of Pharmacy, 19th ed. (1995) Mack Publishing Company, Easton, Pennsylvania).

Mixtures of two or more of the above compounds may be used as desired. The presently preferred enteric coating comprises cellulose acetate phthalate.

The enteric coating material may be mixed with various excipients including plasticizers such as triethyl citrate, acetyl triethyl citrate, diethyl phthalate, dibutyl phthalate, dibutyl subacute, dibutyl tartrate, dibutyl maleate, dibutyl succinate and diethyl succinate and inert fillers such as chalk or pigments.

The composition and thickness of the enteric coating may be selected to dissolve immediately upon contact with the digestive juice of the intestine. Alternatively, the composition and thickness of the exterior coating may be selected to be a time-release coating which dissolves over a selected period of time, as is well known in the art.

The amount of enteric coating depends on the particular enteric coating composition used and is preferably sufficient to substantially prevent the absorption of QDMN or MNTX in the stomach.

Hydroxyalkyl celluloses and their aliphatic esters, carboxyalkyl celluloses and their salts, polycarboxymethylene and its salts and derivatives, polyvinyl alcohol and its esters, polycarboxymethylene copolymer with sodium formaldehyde carboxylates, poly-vinylpyrrolidone, and polyethylene glycol and its esters can be applied as enteric coatings by first dissolving the compound in a minimum amount of water. Alcohol is then added to the point of incipient cloudiness. The mixture can then be applied by conventional techniques.

Application of cellulose acetate phthalate may be accomplished by simply dissolving the cellulose acetate phthalate in a minimum amount of alcohol and then applying by conventional techniques. Hydrogenated vegetable oils may be applied by first dissolving the oil in a minimal amount of a non-polymer solvent, such as methylene chloride, chloroform or carbon tetrachloride, then adding alcohol to the point of incipient cloudiness and then applying by conventional techniques.

In a particularly preferred embodiment, the MNTX is coated with Eudragit L100 or S 1 00, a methacrylic acid copolymer enteric coating, at a 50% coating level to provide stability at gastric pH and dissolution at gut pH per a US Pharmacopeia (USP) standard for enteric coatings.

Any art-known transdermal application may be used, but transdermal administration is preferably via a patch applied to the skin with a membrane of sufficient permeability to allow diffusion of MNTX at a fixed rate in the range of 1.0 to 10.0 mg/hr. The rate of administration may be varied by varying the size of the membrane contact area and/or applying an electrical wiring potential to a drug reservoir. The patch preferably holds 25 mg to 1 gram of available drug in the reservoir plus additional drug as needed for the mechanics of the system.

In the above description, methylnaltrexone is used as an example of a particularly effective QDNM. It is apparent that other QDNM's may be used as desired.

The following Examples are intended to illustrate aspects of the invention and are not to be construed as limitations upon it. The methylnaltrexone used in the following Examples was manufactured by Mallinckrodt Pharmaceuticals, St. Louis, MO. The Enteric Coating was manufactured by Coating Place, Inc., Verona, WI.

### EXAMPLE 1 (REFERENCE)

Ten patients were treated with morphine sulfate administered directly to the central nervous system or intravenously. The morphine sulfate was administered at 0.1 mg/kg body weight. The patients in the study had been treated for pain resulting from surgery. All the patients exhibited pruritus as a side effect of the morphine sulfate administration. Subsequent to the onset of the pruritus, methylnaltrexone, at a dosage of 0.3 mg/kg of body weight was administered intravenously as a saline solution containing methylnaltrexone in a concentration of 5 mg/ml to each of the patients. Eighty percent of the 10 patients exhibited relief from the pruritus sixty minutes after receiving methylnaltrexone.

In a control group, 8 patients were treated with morphine sulfate administered directly to the central nervous system or intravenously. The morphine sulfate was administered at 0.1 mg/kg body weight. The patients in the study had been treated for pain resulting from surgery. All the patients exhibited pruritus as a side effect of the morphine sulfate administration. A placebo, saline at a volume equivalent to the volume administered to patients receiving active drug, was administered intravenously to each of the patients. Only 50% of the patients exhibited relief from the pruritus within sixty minutes.

The study indicates that methylnaltrexone was effective in treating pruritus induced by morphine sulfate.

### EXAMPLE 2 (REFERENCE)

### EFFICACY OF ENTERIC COATING OF METHYLNALTREXONE

Morphine (0.05) mg/kg intravenous) was administered to three volunteers after the oral administration of placebo, methylnaltrexone (6.4 mg/kg) in a gelatin capsule (which dissolves readily in the stomach), or methylnaltrexone after enteric coating (12.8 mg/kg of substance to yield a mass of 6.4 mg/kg methylnaltrexone incorporated) which has decreased release and absorption in the stomach. Oral-cecal transit time was measured using the lactulose-hydrogen breath test. Plasma levels of methylnaltrexone were measured and after the enteric coated preparation were lower. In each subject morphine alone increased the oral-cecai transit time by 20 -70 minutes, methylnaltrexone blocked this effect, and enteric coated methylnaltrexone blocked the effect to a similar or greater extent than the uncoated methylnaltrexone.

### EXAMPLE 3 (REFERENCE)

### ENHANCEMENT OF ENTERIC FEEDING

Two patients receiving morphine (375 mg/day and 18 mg/day) and receiving enteric tube feedings of 200 ml every four (4) hours were studied. The first patient had residual stomach contents of 50cc to 100cc, or 22.0-58.8% of administered feedings measured every 4 hours during a 24 hour control period. Prior to drug administration the residual volume had increased to 260 cc or > 100% of previous feeding volume. Methylnaltrexone, 0.45 mg/kg, was administered intravenously every 4 hours for 24 hours, after the control period. After the first dose (4 hours) of MNTX, the residual was 150cc or 58% of the previous bolus feed, after the 3rd dose (12 hours) the residual was 75cc or 30% of the previous feed, after the 5th dose (20 hours) the residual was 22cc or 13% of the previous feed and after the 6th and final dose (24 hours) the residual was 8cc or 5.5% of previous feed. The follow-up residual sampling after the final drug-tube feed interval had increased to 50cc or 38% or previous feed.

The second patient had greater than 200cc residual or 100% of previous feedings on two consecutive samplings, that is 8 hrs and 4 hrs before drug administration. After initiation of Methylnaltrexone, 0.45 mg/kg, administered intravenously every 4 hours, the first residual (4 hrs) was Occ, the second residual (8 hrs)was 24cc or 15% of previous bolus feed.

### EXAMPLE 4 (REFERENCE)

### TREATMENT OF URINARY RETENTION

Subjects receiving morphine at a variety of doses (via patient controlled analgesia -PCA) who experience urinary retention are administered Methylnaltrexone 0.45 mg/kg intravenously or a placebo. Those treated with Methylnaltrexone have resolution of their symptoms, while those administered placebo go on to require additional therapy (usually urinary catheterization).

### EXAMPLE 5 (REFERENCE)

In a double-blind randomized placebo-controlled study, we evaluated the efficacy of oral methylnaltrexone to decrease subjective effects after administering morphine to 10 normal human volunteers. After intravenous morphine injection (0.05 mg/kg), significant increases in subjective ratings were obtained on "nauseous", "skin itch", "stimulated", and "flushing". Compared to baseline, significant increases were obtained on "nauseous", "Skin itch", "stimulated", and "flushing" ratings after placebo and morphine administration (*P* < 0.05, *P*< 0.05, *P* < 0.01 and *P* < 0.01, respectively). Oral methylnaltrexone (19.2 mg/kg) significantly decreased these four ratings (*P* < 0.05, *P* < 0.05, *P* < 0.01 and *P* < 0.01, respectively) compared to placebo and morphine and resulted in no change when compared to baseline. Plasma methylnaltrexone concentrations were also measured and correlation between pharmacological effects of the compound and its plasma levels was shown. Our results indicate that methylnaltrexone decreases dysphoria and some other undesirable subjective effects associated with opioid medications.

### EXAMPLE 6 (REFERENCE)

### EFFECTS OF ENTERICALLY COATED MNTX ON ORAL-CECAL TRANSIT TIME AND PLASMA LEVELS OF MNTX

Oral methylnaltrexone, whether enterically coated or uncoated, was shown to reverse the inhibitory effects of opioid administration on gastrointestinal motility as measured by oral-cecal transit time. As compared to non-enterically coated MNTX, however, treatment with enterically coated MNTX enhanced the efficacy of the drug at a lower dose while producing lower plasma levels of MNTX.

Subjects were divided into five treatment groups A-E. With the exception of subjects in Group A, who were given a placebo in place of morphine, all were given an intravenous dose of morphine at 0.05 mg/kg. Prior to morphine administration, subjects were given either a placebo or MNTX in various doses and formulations (see Table 1). The subjects in Group A and B were given a placebo in place of MNTX. Group C received uncoated MNTX at 6.4 mg/kg, Group D received enterically coated MNTX at 6.4 mg/kg active drug, and Group E received enterically coated MNTX at 3.2 mg/kg active drug. Table 1 shows the treatments for each group.

**TABLE 1**

| Group | | Treatment combination | FIG. |
|---|---|---|---|
| A | placebo | placebo | |
| B | morphine (0.05 mg/kg) | placebo | |
| C | morphine (0.05 mg/kg) | methylnaltrexone uncoated (6.4 mg/kg) | Fig. 2 |
| D | morphine (0.05 mg/kg) | methylnaltrexone enteric coated (6.4 mg/kg active drug) | Fig. 3 |
| E | morphine (0.05 mg/kg) | methylnaltrexone enteric coated (3.2 mg/kg active drug) | Fig. 4 |
| | | | |

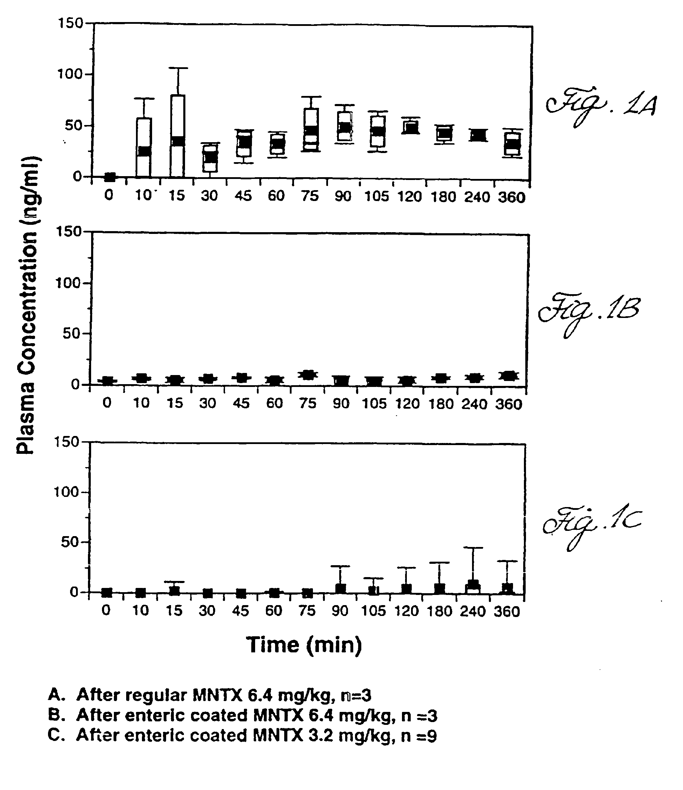

Plasma levels of MNTX were measured following administration of morphine and MNTX or placebo several times over the duration of the six hour monitoring period, at the times shown in FIG. 1. Measurements of plasma and urine MNTX levels were determined by high performance liquid chromatography (HPLC) using the modified method originally reported by Kim et al. (1989) Chromatographia 28:359-63). Methylnaltrexone was separated from plasma by solid phase extraction (SPE). Plasma samples (100-500 µl) diluted in water with the internal standard (naltrexone) were passed through SPE columns. Prior to use, the columns were conditioned by methanol and washed with water. The analytes were eluted from the columns by the mixture of n-propanol and trifluoroacetic acid (25 mM) aqueous solution prepared in 2:1 proportion. The eluate was evaporated to dryness in a stream of nitrogen at 55°C. The residue was reconstituted in the mobile phase, filtered through a nylon HPLC syringe filter and subjected to HPLC analysis. A Shimadzu Corporation (Kyoto, Japan) HPLC system was used. It consisted of the LC-10AD pump, SCL-10A system controller, and SIL-10A auto injector equipped with sample cooler. Used HPLC Analytical Column made by Phenomenex (Prodigy C8, Torrance, CA). The electrochemical detector (ESA Coulochem, model 5100A) worked at the following settings: detector 1, +360 mV, detector 2 +600 mV, guard cell +650 mV. Data were collected with the use of EZChrom 2-2 HPLC software. The mobile phase consisted of 50 mM sodium acetate, 7.5% methanol at pH 4.2. The system was calibrated daily in the range of 5 - 100 ng/ml (3 point calibration). Practical limit of detection for plasma samples was approximately 2 ng/ml (100 pg/injection).

Figure 1 shows the plasma levels of MNTX following the treatments in Groups C, D, and E. In Fig. 1A, MNTX plasma levels in Group C (given 6.4 mg/kg MNTX, uncoated) peaked at about 15 min. post-MNTX administration and remained at a roughly constant level (between about 35-50 ng/ml) for the duration of the study period (6 hours). Group D, given 6.4 mg/kg MNTX in an enterically coated formulation, exhibited a constant low plasma level of MNTX (under 10 ng/ml) for the duration of observation (see FIG. 1B). Group E, given 3.2 mg/kg MNTX in an enterically coated formulation, showed plasma levels of MNTX over the course of observation that were undetectable or at the lower limit of detection of the assay (see FIG. 1C).

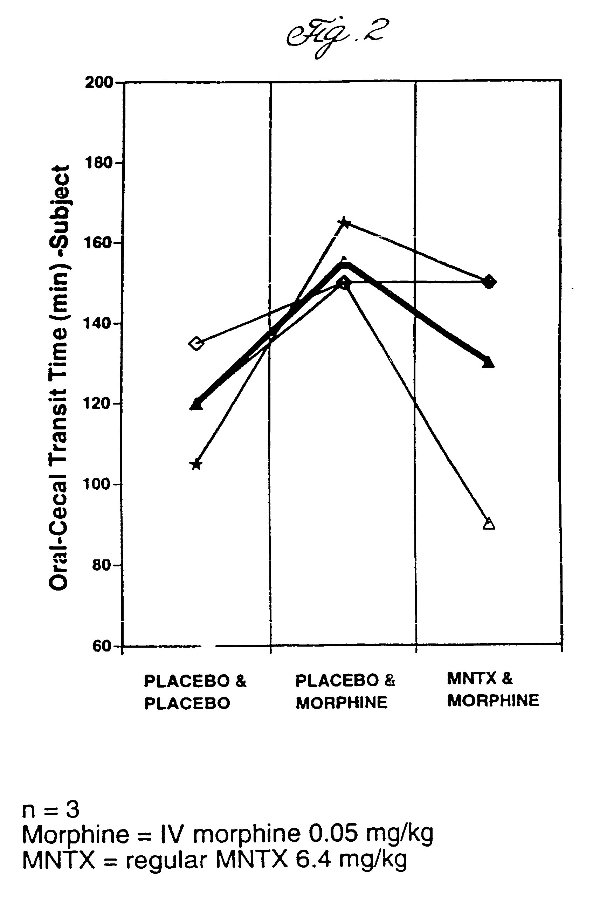

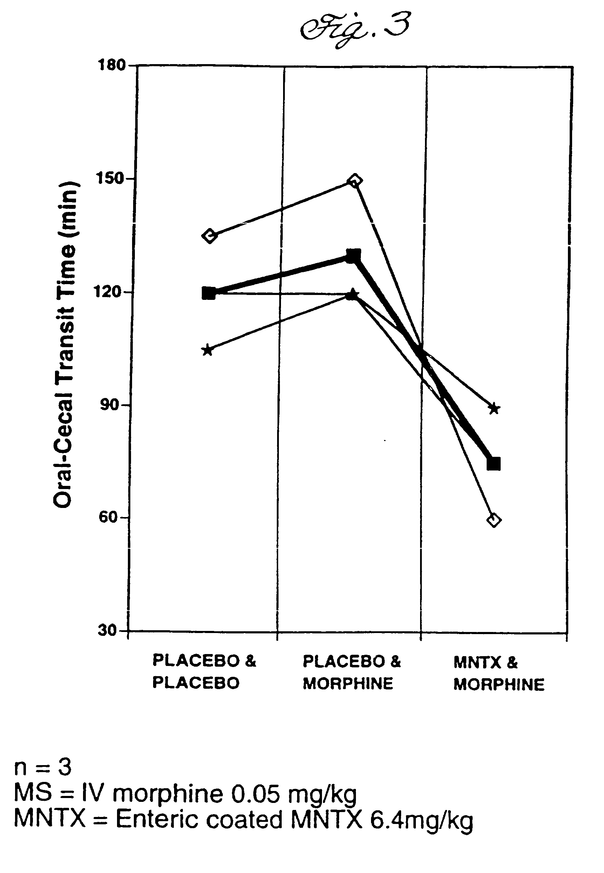

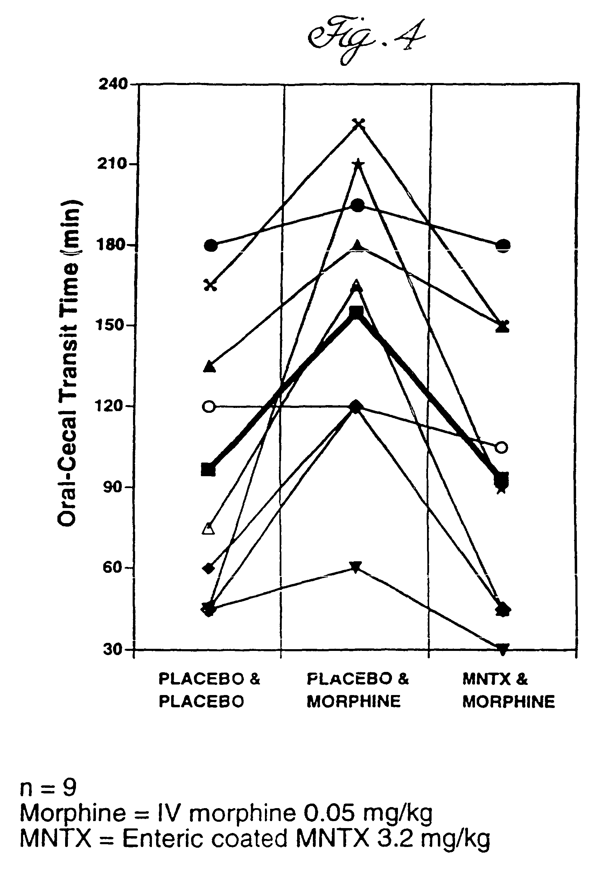

Oral-cecal transit time was used as a measure of gut motility and propensity for constipation. Oral-cecal transit time was measured by the lactulose-breath hydrogen method. Group A demonstrated normal transit times as previously described in the literature (Yuan et al. (1996) Clin. Pharmacol. Ther. 59:469-475; Yuan et al. (1997) Clin. Pharmacol. Ther. 61:467-475). Group B had prolongation of their oral-cecal transit times by 50-100%, while Groups C (FIG. 2) and E (FIG. 4) had their transit times return to baseline levels. Group D showed an obvious decrease in oral-cecal transit time (FIG. 3).

As demonstrated in FIGS. 1-4, enterically coated MNTX provides the therapeutic effects on gastrointestinal motility of uncoated MNTX, but requires a lower dose of active drug and results in significantly reduced plasma levels of MNTX. Patients provided with a dose of 6.4 mg/kg of uncoated MNTX had gut motility return to baseline following morphine administration (FIG. 2) and showed plasma MNTX levels of over 40 ng MNTX/ml, while patients given the same dose in an enterically coated formulation showed oral-cecal transit times below baseline levels (FIG. 3) and plasma MNTX levels under 10 ng/ml. Enterically coated formulations of MNTX with one half the dose of active drug (3.2 mg/kg) were required to return oral-cecal transit times to normal without increasing gut motility. At this dosage, plasma levels of MNTX were negligible.

As with most drugs, it is desirable to maintain the lowest possible systemic levels of MNTX which are sufficient to provide the desired therapeutic effect. For example, elevated circulating levels of MNTX can result in orthostatic hypotension. The present discovery provides an unexpected means to avoid such undesirable drug side effects by lowering the dose administered and subsequently minimizing circulating levels of the drug. Since endogenous and externally supplied opioid-induced inhibition of gastrointestinal motility and constipation is thought to result from opioid receptors located within the gastrointestinal tract, enterically coated MNTX or other QDNMs may provide a local administration of the drug that does not require a circulating level for effective prevention or treatment of symptoms. Thus, the amount and/or frequency of drug administered can be reduced.

## Claims

1. A quaternary derivative of noroxymorphone of the formula: 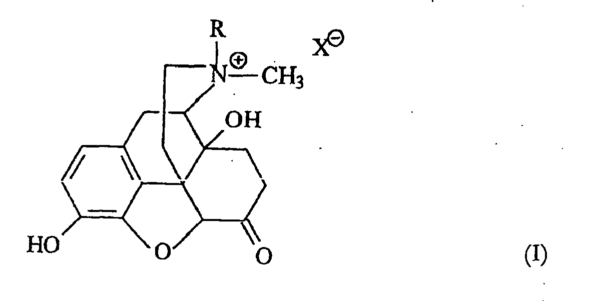 wherein R is allyl, chlorallyl, cyclopropyl-methyl or propargyl, and X is the anion of an acid, for use in treating inhibition of gastrointestinal motility caused by abdominal surgery.

2. A quaternary derivative of noroxymorphone for use as claimed in claim 1, wherein said derivative is to be administered intravenously, intramuscularly, transmucosally, transdermally, or orally.

3. A quaternary derivative of noroxymorphone for use as claimed in claim 2, wherein said derivative is to be administered intravenously, intramuscularly, or transmucosally at a dosage of 0.03 to 1.0mg/kg of body weight.

4. A quaternary derivative of noroxymorphone for use as claimed in claim 2, wherein said derivative is to be administered transdermally via a patch applied to the skin with a membrane of sufficient permeability to allow diffusion of the derivative at a fixed rate in the range of 1.0 to 10 mg/hr.

5. A quaternary derivative of noroxymorphone for use as claimed in claim 2, wherein said derivative is to be administered orally at a dosage of 0.1 to 80mg/kg, or 0.1 to 40.0 mg/kg of body weight.

6. A quaternary derivative of noroxymorphone for use as claimed in claim 1, wherein said derivative is to be administered orally and is formulated as a capsule or a tablet.

7. A quaternary derivative of noroxymorphone for use as claimed in claim 1 or 6 wherein said derivative is enterically coated and to be administered orally.

8. A quaternary derivative of noroxymorphone for use as claimed in any of the preceding claims, wherein said derivative is to be administered prior to the onset of gastrointestinal motility inhibition.

9. A quaternary derivative of noroxymorphon for use as claimed in any of the preceding claims, wherein said derivative is to be administered after the onset of gastrointestinal motility inhibition.

10. A quaternary derivative of noroxymorphone for use as claimed in any of the preceding claims, wherein X is a chloride, bromide, iodide or methylsulfate anion.

11. A quaternary derivative of noroxymorphone for use as claimed in any of the preceding claims, wherein said derivative is methylnaltrexone.

12. Use of a quaternary derivative of noroxymorphone as defined in any of claims 1, 10 or 11 for the manufacture of a medicament for use in treating inhibition of gastrointestinal motility caused by abdominal surgery.

## Patentansprüche

1. Quaternäres Derivat von Noroxymorphon der Formel: 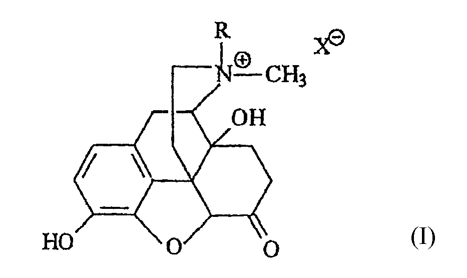 worin R für Allyl, Chlorallyl, Cyclopropylmethyl oder Propargyl steht, und X für das Anion einer Säure steht, zur Anwendung in der Behandlung der Hemmung der durch Abdominalchirurgie verursachten gastrointestinalen Motilität.

2. Quaternäres Derivat von Noroxymorphon zur Anwendung nach Anspruch 1, worin das Derivat intravenös, intramuskulär, transmukosal, transdermal oder oral zu verabreichen ist.

3. Quaternäres Derivat von Noroxymorphon zur Anwendung nach Anspruch 2, worin das Derivat intravenös, intramuskulär oder transmukosal in einer Dosierung von 0,03 bis 1,0 mg/kg Körpergewicht zu verabreichen ist.

4. Quaternäres Derivat von Noroxymorphon zur Anwendung nach Anspruch 2, worin das Derivat über ein auf die Haut aufzubringendes Pflaster mit einer Membran von ausreichender Permeabilität transdermal zu verabreichen ist, um die Diffusion des Derivats bei einer fixen Rate im Bereich von 1,0 bis 10 mg/h zu erlauben.

5. Quaternäres Derivat von Noroxymorphon zur Anwendung nach Anspruch 2, worin das Derivat oral in einer Dosierung von 0,1 bis 80 mg/kg oder 0,1 bis 40,0 mg/kg Körpergewicht zu verabreichen ist.

6. Quaternäres Derivat von Noroxymorphon zur Anwendung nach Anspruch 1, worin das Derivat oral zu verabreichen ist und als eine Kapsel oder eine Tablette formuliert ist.

7. Quaternäres Derivat von Noroxymorphon zur Anwendung nach den Ansprüchen 1 oder 6, worin das Derivat magensaftresistent überzogen ist und oral zu verabreichen ist.

8. Quaternäres Derivat von Noroxymorphon zur Anwendung nach einem der vorangehenden Ansprüche, worin das Derivat vor dem Einsetzen der gastrointestinalen Motilitätshemmung zu verabreichen ist.

9. Quaternäres Derivat von Noroxymorphon zur Anwendung nach einem der vorangehenden Ansprüche, worin das Derivat nach dem Einsetzen der gastrointestinalen Motilitätshemmung zu verabreichen ist.

10. Quaternäres Derivat von Noroxymorphon zur Anwendung nach einem der vorangehenden Ansprüche, worin X für ein Chlorid-, Bromid-, Iodid- oder Methylsulfatanion steht.

11. Quaternäres Derivat von Noroxymorphon zur Anwendung nach einem der vorangehenden Ansprüche, worin das Derivat Methylnaltrexon ist.

12. Verwendung eines quaternären Derivats von Noroxymorphon nach einem der Ansprüche 1, 10 oder 11 zur Herstellung eines Arzneimittels zur Anwendung in der Behandlung der Hemmung der durch Abdominalchirurgie verursachten gastrointestinalen Motilität.

## Revendications

1. Dérivé quaternaire de noroxymorphone de formule : 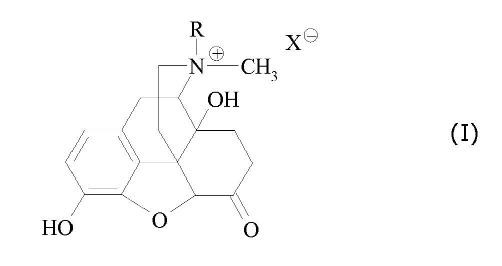 dans laquelle R est un groupe allyle, chloroallyle, cyclopropylméthyle ou propargyle, et X est l'anion d'un acide, destiné à être utilisé dans le traitement de l'inhibition de la motilité gastro-intestinale consécutive à une opération de chirurgie abdominale.

2. Dérivé quaternaire de noroxymorphone destiné à être utilisé selon la revendication 1, dans lequel ledit dérivé doit être administré par voie intraveineuse, intramusculaire, transmucosale, percutanée ou orale.

3. Dérivé quaternaire de noroxymorphone destiné à être utilisé selon la revendication 2, dans lequel ledit dérivé doit être administré par voie intraveineuse, intramusculaire ou transmucosale en une dose allant de 0,03 à 1,0 mg par kg de masse corporelle.

4. Dérivé quaternaire de noroxymorphone destiné à être utilisé selon la revendication 2, dans lequel ledit dérivé doit être administré par voie percutanée au moyen d'un timbre, appliqué sur la peau, qui possède une membrane dont la perméabilité est suffisante pour permettre la diffusion du dérivé à un débit fixe dans la plage de 1,0 à 10 mg/h.

5. Dérivé quaternaire de noroxymorphone destiné à être utilisé selon la revendication 2, dans lequel ledit dérivé doit être administré par voie orale en une dose allant de 0,1 à 80 mg ou de 0,1 à 40,0 mg par kg de masse corporelle.

6. Dérivé quaternaire de noroxymorphone destiné à être utilisé selon la revendication 1, dans lequel ledit dérivé doit être administré par voie orale et est formulé sous la forme d'une gélule ou d'un comprimé.

7. Dérivé quaternaire de noroxymorphone destiné à être utilisé selon la revendication 1 ou la revendication 6, dans lequel ledit dérivé est doté d'un enrobage entérique et doit être administré par voie orale.

8. Dérivé quaternaire de noroxymorphone destiné à être utilisé selon l'une quelconque des revendications précédentes, dans lequel ledit dérivé doit être administré avant l'apparition de l'inhibition de la motilité gastro-intestinale.

9. Dérivé quaternaire de noroxymorphone destiné à être utilisé selon l'une quelconque des revendications précédentes, dans lequel ledit dérivé doit être administré après l'apparition de l'inhibition de la motilité gastro-intestinale.

10. Dérivé quaternaire de noroxymorphone destiné à être utilisé selon l'une quelconque des revendications précédentes, dans lequel X est un anion chlorure, bromure, iodure ou méthylsulfate.

11. Dérivé quaternaire de noroxymorphone destiné à être utilisé selon l'une quelconque des revendications précédentes, dans lequel ledit dérivé est la méthylnaltrexone.

12. Utilisation d'un dérivé quaternaire de noroxymorphone selon l'une quelconque des revendications 1, 10 ou 11 pour fabriquer un médicament destiné à être utilisé dans le traitement de l'inhibition de la motilité gastro-intestinale consécutive à une opération de chirurgie abdominale.
